# EUROPÄISCHE PATENTSCHRIFT

(11) **EP 2 024 278 B1**
(45) Veröffentlichungstag und Bekanntmachung des Hinweises auf die Patenterteilung: **28.10.2009**
(21) Anmeldenummer: 07724818.5
(22) Anmeldetag: 03.05.2007
(51) Int. Cl.: C01B 7/04, C01B 7/07, C01B 17/88, C01B 17/94, C01B 31/28, C25B 1/26, C07C 263/10, C07C 201/08, C07C 205/06

(54) **VERFAHREN ZUR GEKOPPELTEN HERSTELLUNG VON CHLOR UND ISOCYANATEN**
METHOD FOR THE COUPLED PRODUCTION OF CHLORINE AND ISOCYANATES
PROCÉDÉ POUR LA PRÉPARATION CONJUGUÉE DE CHLORE ET D'ISOCYANATES

(30) Priorität: 13.05.2006 DE 102006022447
(43) Veröffentlichungstag der Anmeldung: 18.02.2009
(73) Patentinhaber: Bayer MaterialScience AG, 51368 Leverkusen (DE)
(72) Erfinder: DUGAL, Markus, 47906 Kempen (DE); PENNEMANN, Bernd, 51467 Bergisch Gladbach (DE); KÄMPER, Friedhelm, 47807 Krefeld (DE)
(74) Vertreter: Klimiuk, Meike
(86) Internationale Anmeldenummer: PCT/EP2007/003892
(87) Internationale Veröffentlichungsnummer: WO 2007/131638

(56) Entgegenhaltungen:
- DE-A1- 2 063 592
- DE-A1- 19 642 328
- US-A- 3 201 201
- US-A- 5 888 920
- US-A- 6 156 288
- EVANS C: "CONCENTRATION AND RECOVERY OF NITRATION SPENT ACIDS" SULPHUR, BRITISH SULPHUR PUBLISHING, LONDON, GB, Nr. 246, 1. September 1996 (1996-09-01), Seiten 51-53,55,57, XP000626948 ISSN: 0039-4890

## Beschreibung

Die vorliegende Erfindung betrifft ein Verfahren zur gekoppelten Herstellung von Chlor und Isocyanaten, wobei die für beide Verfahren eingesetzte Schwefelsäure nach Einsatz vereint wird, gemeinsam aufkonzentriert wird und in eines oder beide Verfahren wieder zurückfließt.

Chlor und Nitroaromaten sind wichtige industrielle Zwischenprodukte, die beispielsweise für die Herstellung von Polyurethanrohstoffen wie z.B. Toluylendiisocyanat (TDI) oder Methylendiphenyldiisocyanat (MDI) benötigt werden. Chlorgas wird dabei zur Herstellung von Phosgen benötigt, das zur Herstellung von Isocyanaten aus Aminen eingesetzt wird, wobei der dabei als Nebenprodukt entstehende Chlorwasserstoff (HCl) nach verschiedenen Recycling-Verfahren wieder zu Chlor umgesetzt werden kann. Chlor ist jedoch auch aus anderen Rohstoffen wie z.B. Natriumchlorid über Elektrolyseverfahren herstellbar.

Für die Produktion von Chlor werden hier beispielhaft die folgenden verschiedenen großtechnischen Verfahren erwähnt:
1. Die Produktion von Chlor in NaCl-Elektrolysen
2. Die Umwandlung von HCl zu Chlor durch Elektrolyse von wässriger HCl mit Diaphragmen oder Membranen als Trennmedium zwischen Anoden- und Kathodenraum. Das Nebenprodukt ist hierbei Wasserstoff.
3. Die Umwandlung von HCl zu Chlor durch Elektrolyse von wässriger HCl in Gegenwart von Sauerstoff in Elektrolysezellen mit Sauerstoff-Verzehr-Kathode (ODC, Oxygen Depletion Cathode). Das Nebenprodukt ist hierbei Wasser.
4. Die Umwandlung von HCl-Gas zu Chlor durch Gasphasenoxidation von HCl mit Sauerstoff bei erhöhten Temperaturen an einem Katalysator. Das Nebenprodukt ist hierbei ebenfalls Wasser. Dieses Verfahren ist als "Deacon Verfahren" seit über einem Jahrhundert bekannt und in Benutzung.

Bei den meisten Verfahren zur Chlorherstellung sowohl aus wässriger wie aus gasförmigem HCl wird das Prozessgas nach der Reaktion und gegebenenfalls ersten Aufarbeitungsschritten wie beispielsweise der Absorption von nicht umgesetzter HCl einem Trocknungsschritt unterzogen, um gegebenenfalls enthaltenes Wasser, welches in nachfolgenden Aufarbeitungsschritten, wie beispielsweise der Chlorverflüssigung oder Destillation, oder Anwendungen störend sein kann, zu entfernen. Hierzu wird in der Regel konzentrierte Schwefelsäure im Bereich von 90,0-98,0 % Massenanteil Schwefelsäure bezogen auf Massenanteil Schwefelsäure und Wasser als Trocknungsmittel verwendet. Es entstehen dabei große Mengen an verdünnter Schwefelsäure mit einer Konzentration von 70,0 bis 89,9 % Massenanteile Schwefelsäure bezogen auf Massenanteile Schwefelsäure und Wasser, die entweder unter Energieaufwand in einer Destillationsanlage aufkonzentriert oder entsorgt werden muss.

JP 2004-269408 und JP 2001-192647 beschreiben Methoden zur Chlorgastrocknung mit Schwefelsäure in Trocknungskolonnen unter Minimierung von Schwefelsäureverlusten und Rezyklisierung von Teilströmen gebrauchter Schwefelsäure durch Vermischung mit hochkonzentrierter frischer Schwefelsäure, ohne dass jedoch eine destillative Aufkonzentrierung der gebrauchten Schwefelsäure beschrieben wird. Des Weiteren beschreiben diese beiden Dokumente nicht, dass die konzentrierte oder verdünnte Schwefelsäure aus dem Prozess auch Bestandteil der Nitriersäure in einer Nitrierungsreaktion sein kann.

US 3,201,201 beschreibt die Trocknung von Chlor-Gas aus der HCl-Oxidation und die Regeneration der Schwefelsäure durch adiabate Flashverdampfung. Auch hier wird ein weiterer Einsatz der Schwefelsäure in einer Nitrierungsreaktion mit Nitriersäure nicht beschrieben.

Einfach oder zweifach nitrierte aromatische Verbindungen ausgewählt aus der Gruppe von Nitrobenzol, Dinitrobenzol, Nitrochlorbenzol, Nitrochlortoluol, Nitrotoluol und Dinitrotoluol werden, abgesehen von wenigen Ausnahmen, in der Regel über Nitrierverfahren hergestellt, bei denen die aromatische Ausgangsverbindung ausgewählt aus der Gruppe von Benzol, Chlorbenzol und Toluol in einer Schwefelsäure-katalysierten Zweiphasenreaktion mit Salpetersäure zur gewünschten einfach oder zweifach nitrierten aromatischen Verbindung umgesetzt wird. Dabei kommen verschiedenste Verfahrensvarianten wie z.B. die isotherme oder die adiabate Nitrierung zum Einsatz. Eine Übersicht der verschiedenen gängigen Verfahrens- und Reaktortypen ist beispielsweise in EP-A 0708076 beschrieben.

Die Schwefelsäure muss für den Wiedereinsatz nach der Reaktion vom nitrierten Produkt getrennt und beispielsweise durch Destillation unter Energieaufwand aufkonzentriert werden Die Aufkonzentrierung der aus der Reaktion ablaufenden Schwefelsäure kann je nach gewünschter Zielkonzentration z.B. durch eine mehrstufige Vakuumdestillation wie sie in US 6,156,288 und DE-A 19 642 328 beschrieben ist oder durch Flashverdampfung, wie sie in US 3201201 beschrieben ist, erfolgen.

Nach den gängigen Verfahren zur Chlortrocknung gemäß dem beschriebenen Stand der Technik entstehen also große Mengen an verdünnter Schwefelsäure, die entweder unter hohem Energieaufwand in einer Destillationsanlage aufkonzentriert oder über das Abwasser der Chlorherstellung entsorgt werden muss. Bei der Nitrierung von Aromaten wird die als Katalysator eingesetzte Schwefelsäure zum Wiedereinsatz nach der Reaktion ebenfalls unter hohem Energieaufwand aufkonzentriert. Bei diesem Verfahren gehen zudem große Mengen Schwefelsäure über den Produkt/Abwasserweg verloren, die durch frische Schwefelsäure ersetzt werden muss. Die im Abwasser des Chlorherstellungs- und des Nitrierverfahrens enthaltene Schwefelsäure muss unter Einsatz großer Mengen an Natronlauge neutralisiert werden, bevor das Abwasser einer Kläranlage zugeführt werden kann.

US 5,888,920 beschreibt zwar, dass die bei der Alkylierung, der Chlortrocknung oder der Nitrierung anfallende verdünnte Schwefelsäure nach einer entsprechenden Aufkonzentrierung wieder in die jeweiligen Reaktionsverfahren aus denen sie erhalten wurden, zurückgeführt werden können, US 5,888,920 beschreibt jedoch nicht, dass eine verdünnte Schwefelsäure, die aus einem Nitrier- oder Chlortrocknungsverfahren gewonnen wurde nach Vereinigung dieser zwei Schwefelsäuren und nach Aufkonzentrierung wieder in einen der beiden oder in beide zurückgeführt werden kann, ohne dass dabei darauf geachtet werden müsste, aus welchem Verfahren die ursprüngliche verdünnte Schwefelsäure erhalten wurde.

Die Aufgabe der vorliegenden Erfindung ist es daher ein Verfahren zur gekoppelten Herstellung von Chlor und Isocyanaten bereit zustellen, das es ermöglicht die bei beiden Verfahren anfallende verdünnte Schwefelsäure ohne hohe Energiekosten und Investitionskosten und unter Einsparung von Neutralisationsmitteln in einem gemeinsamen Verfahren so aufzuarbeiten, dass sie sowohl in die Chlorherstellung als auch in die Nitrierreaktionen zurückgeführt werden kann, unabhängig davon aus welchem Verfahren die verdünnte Schwefelsäure ursprünglich erhalten wurde.

Diese Aufgabe wird gelöst durch ein Verfahren zur gekoppelten Herstellung von Isocyanaten und Chlor umfassend folgende Schritte
a) Nitrierung von aromatischen Verbindungen ausgewählt aus der Gruppe von Toluol, halogeniertem Toluol, Benzol und halogeniertem Benzol zu den entsprechenden einfach oder zweifach nitrierten aromatischen Verbindungen unter Einsatz von Salpetersäure, wobei bevorzugt die Nitrierung jeder einzelnen aromatischen Verbindung in voneinander unabhängigen und nicht mit einander verbundenen Reaktoren stattfindet und die Nitrierung in Abhängigkeit von der zu nitrierenden Verbindung in Gegenwart einer Schwefelsäure mit einer Konzentration von 65,0 - 98,0 % Massenanteile Schwefelsäure bezogen auf Massenanteile Schwefelsäure und Wasser als Katalysator durchgeführt wird, wobei die Schwefelsäure nach erfolgter Nitrierung verdünnt ist und eine Konzentration aufweist, die je nach eingesetzter Schwefelsäuremenge und Zahl der Nitrierstufen um 0,5 bis 25 Prozentpunkte niedriger ist als die jeweils eingesetzte Schwefelsäure, d. h. eine Konzentration von 40 - 97,5 Gew.-% Schwefelsäure, bezogen auf das Gewicht von Schwefelsäure und Wasser, hat.
b) Überführung der aus dem Schritt a) erhaltenen verdünnten Schwefelsäure in eine Schwefelsäureaufkonzentrieranlage,
c1) Umsetzung der aus Schritt a) erhaltenen einfach oder zweifach nitrierten aromatischen Verbindung mit Wasserstoff zu dem entsprechenden Amin, oder
c2) für den Fall, dass die einfach oder zweifach nitrierte Verbindung Nitrobezol ist, Umsetzung des aus Schritt a) erhaltenen Nitrobenzols mit Wasserstoff zu dem entsprechendem Anilin und anschließende Umsetzung dieses Produktes mit Formaldehyd zu den Di- und Polyaminen der Diphenylmethanreihe,
d) Umsetzung der Amine erhältlich aus Schritt c1) oder c2) mit Phosgen zu den entsprechenden Isocyanaten,
e) Rückführung des bei der Reaktion der Amine aus Schritt c1) oder c2) mit Phosgen entstehenden wässrig gelösten oder gasförmigen Chlorwasserstoffs aus Schritt d) in Schritt f);
f) Herstellung von Chlorgas
g) Entfernung des Reaktionswassers oder aus anderen Quellen enthaltene Feuchtigkeit unter Trocknung aus dem aus Schritt f) erhaltenen Chlorgas durch Behandlung des aus Schritt f) erhaltenen Chlorgases mit einer Schwefelsäure mit einer Konzentration von 90,0 bis 99,0 % Massenanteile Schwefelsäure bezogen auf Massenanteile Schwefelsäure und Wasser bis die Schwefelsäure eine Konzentration von 65,0-90,0 % Massenanteile Schwefelsäure bezogen auf Massenanteile Schwefelsäure und Wasser aufweist,
h) Umsetzung des aus Schritt g) erhaltenen, trockenen Chlorgases mit Kohlenmonoxid zur Herstellung von Phosgen,
i) Einsatz des aus Schritt h) erhaltenen Phosgens in den Schritt d),
j) Überführung der aus Schritt g) erhaltenen verdünnten Schwefelsäure entweder in die gleiche Schwefelsäureaufkconzentrieranlage von Schritt b) oder direkt in einen oder mehrere Nitrierschritte gemäß Schritt a) und anschließende Überführung der aus dieser oder diesen Nitrierungen ablaufenden, weiter verdünnten Schwefelsäuren in die gleiche Schwefelsäureaufkonzentrieranlage gemäß Schritt b),
   wobei die verdünnten Schwefelsäureströme aus Schritt b) und g) in der Schwefelsäureaufkonzentrieranlage vereint werden und durch eine oder mehrere Vakuumdestillationsstufen auf eine erhöhte Konzentration oder durch Abnahme von Teilströmen nach verschiedenen Destillationsstufen auf mehrere verschiedene erhöhte Konzentrationen angehoben wird, wie sie jeweils für einen oder mehrere der Verfahrenschritte der Aromatennitrierung nach Schritt a) oder Chlorgastrocknung nach Schritt g) benötigt werden
k) und anschließende Rückführung der aus Schritt j) gewonnenen aufkonzentrierten Schwefelsäure entweder zum vollständigem oder teilweisen Einsatz in Schritt g) und / oder Schritt a).

Vorteilhaft ist das erfindungsgemäße Verfahren wenn die Herstellung des Chlorgas gemäß Schritt f) nach dem erfindungsgemäßen Verfahren nach einem oder mehreren der Verfahren ausgewählt aus der Gruppe von NaCI-Elektrolyse, HCl-Elektrolyse nach Membran- oder Diaphragma- Verfahren, HCl-Elektrolyse in Elektrolysezellen mit Sauerstoffverzehrkathode und katalytische HCl-Oxidation mit Sauerstoff erfolgt.

Vorteilhaft ist das erfindungsgemäße Verfahren, wenn die einfach oder zweifach nitrierten aromatischen Verbindungen ausgewählt sind aus der Gruppe von Nitrobenzol, Dinitrobenzol, Nitrochlorbenzol, Nitrochlortoluol, Nitrotoluol und Dinitrotoluol.

Vorteilhaft ist das erfindungsgemäße Verfahren, wenn die verdünnte Schwefelsäure, die nach dem Verfahrensschritt g) erhalten wird vor Vereinigung in der Schwefelsäureaufkonzentrierungsanlage gemäß Schritt j) von Chlor- und/ oder HCl-Resten bis auf eine Restkonzentration von < 1000ppm Cl befreit wird.

Vorteilhaft ist das erfindungsgemäße Verfahren, wenn die verdünnte Schwefelsäure, die nach dem Verfahrensschritt g) erhalten wird vor Vereinigung in der Schwefelsäureaufkonzentrierungsanlage gemäß Schritt j) von Chlor- und/ oder HCl- Resten bis auf eine Restkonzentration von < 10 ppm Cl befreit wird.

Vorteilhaft ist das erfindungsgemäße Verfahren, wenn entweder bereits die verdünnte Schwefelsäure erhältlich nach Schritt a) oder erst die aufkonzentrierte Schwefelsäure erhältlich nach Schritt j) des erfindungsgemäßen Verfahrens vor Rücküberführung in Schritt k) des erfindungsgemäßen Verfahrens von Verunreinigungen durch anorganische Stickstoffverbindungen wie Nitrosylschwefelsäure bis auf eine Restkonzentration von < 0,3 Gew.- % oder durch leichtflüchtige organische Verbindungen wie Dinitrotoluol, Nitrobenzol, Dinitrobenzol, Nitrophenole, Nitrokresole, Nitrobenzylalkohole, Nitrobenzaldehyde bis auf eine Restkonzentration von < 50 ppm, oder durch schwer flüchtige organische Verbindungen wie Hydroxynitrobenzoesäure, Nitrobenzoesäuren oder aliphatische Carbonsäuren bis auf eine Restkonzentration von < 500 ppm oder durch flüchtige Schwefelverbindungen wie SO₂ bis auf eine Restkonzentration von < 30 ppm befreit wird.

Vorteilhaft ist das erfindungsgemäße Verfahren, wenn in Schritt a) Toluol eingesetzt wird und nach zweifacher Nitrierung in Schritt a) Dinitrotoluol erhalten wird, wobei im ersten Schritt der Nitrierung eine Schwefelsäure mit einer Konzentration von 86,0-96,0 % Massenanteile Schwefelsäure bezogen auf die Summe der Massenanteile von Schwefelsäure und Wasser, eingesetzt wird, die im Laufe der zweiten Nitrierungsstufe auf eine Konzentration von 80,0-85,9 % Massenanteile Schwefelsäure bezogen auf die Summe der Massenanteile von Schwefelsäure und Wasser, verdünnt wird, das Dinitrotoulol anschließend gemäß Schritt c1) mit Wasserstoff zu Toluylendiamin (TDA) umgesetzt wird, welches anschließend mit Phosgen gemäß Schritt d) zu Toluylendiisocyanat (TDI) umgesetzt wird.

Vorteilhaft ist das erfindungsgemäße Verfahren, wenn die Schwefelsäure, die aus der zweiten Nitrierstufe mit einer Konzentration von 80,0-85,9 % Massenanteile Schwefelsäure bezogen auf die Masseanteile an Schwefelsäure und Wasser, erhalten wird, noch einmal in die erste Nitrierstufe des Verfahrens nach Schritt a) zurückgeleitet wird und anschließend auf eine Konzentration von 70,0-79,9 % Massenanteile Schwefelsäure bezogen auf die Masseanteile Schwefelsäure und Wasser, verdünnt wird und diese Schwefelsäure anschließend in die Schwefelsäureaufkonzentrierungsanlage gemäß Schritt b) geleitet wird.

Vorteilhaft ist das erfindungsgemäße Verfahren, wenn in Schritt a) Benzol eingesetzt wird und nach einfacher Nitrierung in Schritt a) Nitrobenzol erhalten wird, wobei zur Nitrierung eine Schwefelsäure mit einer Konzentration von 69,5-72,5 % Massenanteile Schwefelsäure bezogen auf die Masseanteile Schwefelsäure und Wasser, eingesetzt wird, die im Laufe der Nitrierung auf eine Konzentration von 66,5-69,4 % Massenanteile Schwefelsäure bezogen auf die Masseanteile Schwefelsäure und Wasser, verdünnt wird, das Nitrobenzol anschließend gemäß Schritt c2) mit Wasserstoff zu Anilin umgesetzt wird, welches wiederum mit Formaldehyd in Gegenwart eines sauren Katalysators zu Di- und Polyaminen der Diphenylmethanreihe umgesetzt wird, welche wiederum anschließend mit Phosgen gemäß Schritt d) zu den entsprechenden Di- und Polyisocyanaten der Diphenylmethanreihe umgesetzt werden.

Für den Teil der Chlorherstellung in dem erfindungsgemäßen Verfahren können alle dem Fachmann bekannten Chlorerzeugungsverfahren, wie z.B. die Produktion von Chlor durch NaCl-Elektrolyse, die Umwandlung von HCl zu Chlor durch Elektrolyse von wässriger HCl mit Diaphragmen oder Membranen als Trennmedium zwischen Anoden- und Kathodenraum, die Umwandlung von HCl zu Chlor durch Elektrolyse von wässriger HCl in Gegenwart von Sauerstoff in Elektrolysezellen mit Sauerstoff-Verzehr-Kathode (ODC, Oxygen Depletion Cathode) und die Umwandlung von HCl-Gas zu Chlor durch Gasphasenoxidation von HCl mit Sauerstoff bei erhöhten Temperaturen an einem Katalysator zum Einsatz kommen.

Besonders bevorzugt ist die Herstellung von Chlor durch Umsetzung von HCl-Gas durch Gasphasenoxidation mit Sauerstoff bei Temperaturen im Bereich von 180°C bis 500°C in einem Deacon-Prozess oder einem vom Deacon-Prozess abgeleiteten Verfahren (wie z.B. in EP-743277-B1, DE 19734412-A1, DE 19748299-A1, DE 10242400; DE 10244996 beschrieben) an einem ortsfesten Katalysator enthaltend mindestens ein Metall in elementarer oder gebundener Form ausgewählt aus der Gruppe Cu, Ru, Au, Rh, Pd, Pt, Os, Ir, Ag, Re Ce, Bi, Ni, Co, Ga, Fe und Nd wobei die Metalle oder die Metallverbindungen ungeträgert oder geträgert auf z.B. Metall- oder Halbmetalloxiden wie z.B. Alumniumoxide, Siliziumoxide, Titanoxide, Zirkonoxide oder Zinnoxide, auf Mischmetalloxiden, auf keramischen Materialien, auf Aktivkohlen, Rußen oder Carbon-Nanotubes, auf Metall-oder Halbmetallcarbiden, auf Metall- oder Halbmetallnitriden oder auf Metall- oder Halbmetallsulfiden vorliegen können.

Bei Einsatz der katalytischen Gasphasenoxidation zur Umsetzung des in Schritt e) entstehenden Chlorwasserstoffs wird in der Regel der gasförmige Chlorwasserstoff aus dem Phosgenierverfahren in das HCl-Oxidationsverfahren überführt. Optional können vor der Durchführung der Gasphasenoxidation mit dem Chlorwasserstoffgas Reinigungsoperationen z.B. zur Entfernung von Phosgen, von Lösungsmittelresten, wie z.B. Chlorbenzol oder o-Dichlorbenzol, von Chlorkohlenwasserstoffen, von organischen und anorganischen Schwefel- oder Stickstoffverbindungen oder von Kohlenstoffoxiden wie CO oder CO₂ erfolgen. Mögliche anwendbare Trenn- oder Reinigungsoperationen sind hierbei z.B. Adsorption, Absorption-Desorption, Tiefkälte-Kondensation, Kompression, Druckdestillation oder selektive Oxidation. Die katalytische Gasphasenoxidation und Aufarbeitung des Produktgasstroms kann in verschiedensten in der Literatur beschriebenen Varianten wie beispielsweise in EP-B1 743277, US 6,852,667, DE-A 19734412, WO 2005014470, DE-A 10244996, US-A 200411411 und EP-B 767138 beschrieben, durchgeführt werden.

Das im erfindungsgemäßen Verfahren enthaltene Nitrierverfahren gemäß Schritt a) kann zur Herstellung der verschiedensten Nitroaromaten ausgewählt aus der Gruppe von Nitrobenzol, Dinitrobenzol, Nitrochlorbenzol, Nitrochlortoluol, Nitrotoluol und Dinitrotoluol eingesetzt werden. Dabei sind alle dem Fachmann für das jeweilig angestrebte Zielprodukt geläufigen Verfahren zur Schwefelsäure-katalysierten Nitrierung, wie beispielsweise in EP-A 0708076 beschrieben wird, einsetzbar.

Auch eine Kopplung einer oder mehrerer verschiedener Chlorherstellungsverfahren mit einem oder mehreren Nitroarornatenherstellverfahren bzw. Isocyanatherstellungsverfahren ist möglich.

Die Aufkonzentrierung der Säure kann nach allen gemäß dem Stand der Technik gängigen Verfahren zur Wasserentfernung aus Schwefelsäure geschehen. Dies sind beispielsweise ein- oder mehrstufige Vakuumdestillation oder Flashverdampfung, Erhitzung mit Mikrowellen oder Elektromembrantechniken. Besonders bevorzugt ist jedoch der Einsatz von ein- oder mehrstufigen Vakuumdestillationen und/oder Flashverdampfungen.

In einer speziellen Ausführungsform des erfindungsgemäßen Verfahrens wird konzentrierte Schwefelsäure im Bereich von 90,0-98,0 % Massenanteile Schwefelsäure bezogen auf Massenanteile Schwefelsäure und Wasser für die Chlorgastrocknung verwendet und die verdünnte Schwefelsäure im Bereich, von 70,0-90,0 % Massenanteile Schwefelsäure bezogen auf Massenanteile Schwefelsäure und Wasser aus der Chlorgastrocknung in eine Schwefelsäureaufkonzentrierungseinheit einer Nitrieranlage z.B. zur Dinitrierung von Toluol geleitet. Die Menge der dem Nitrier/Säurekonzentrationsverfahren zugeführten Säure entspricht dabei ganz oder zum Teil den Schwefelsäureverlusten des Nitrier/Säurekonzentrationsverfahrens.

In einer weiteren alternativen Fahrweise kann die Schwefelsäure aus dem Chlortrocknungsprozess auch direkt in eine oder mehrere Reaktionsstufen des Nitrierverfahrens eingeleitet werden und als Katalysator für die Nitrierreaktion verwendet werden, wobei die dabei entstehende verdünnte Schwefelsäure anschließend einer Säureaufkonzentrierung zugeführt wird. Auf diese Weise kann durch Kombination der Verfahren sowohl eine Entsorgung und die damit verbundene Neutralisation mit Natronlauge oder eine gesonderte Aufbereitung der Schwefelsäure aus der Chlorgastrocknung eingespart als auch die Zukaufmenge an frischer Schwefelsäure für das Nitrierverfahren minimiert werden.

Verunreinigungen in der verdünnten Schwefelsäure aus dem Chlorherstellungsverfahren nach Schritt g) des erfindungsgemäßen Verfahrens wie beispielsweise Chlorreste können gegebenenfalls vor Einleiten in eine Schwefelsäureaufkonzentrierungsanlage einer Nitrieranlage oder eines Nitrierprozesses abgetrennt werden, um Korrosion und Chlorkontamination im Nitrierverfahren zu vermeiden. Diese Aufreinigung der Schwefelsäure kann durch verschiedenste Verfahren wie es beispielsweise in DE-A 2 063 592 durch Adsorption an Aktivkohle beschrieben ist oder wie es beispielsweise in Khorasani et. al., Journal of Bangladesh Academy of Sciences, 1982, 6 (1-2), 205-207 durch Strippverfahren mit Luft beschrieben ist, erfolgen. Zur Vermeidung von Korrosionsproblemen können auch resistente Materialien wie spezielle Nickel-Legierungen, die in JP 2006045610 offenbart sind, in der Schwefelsäureaufkonzentrierungsanlage verwendet werden. Bevorzugt ist eine Entfernung von Chlorresten über Strippverfahren. Die Schwefelsäure wird hierbei von Chlor und HCl- Rest befreit, so dass eine Restkonzentration an Cl im Bereich von < 1000 ppm, bevorzugt kleiner 100 ppm, besonders bevorzugt kleiner 10 ppm erhalten wird.

In einer weiteren Ausführungsform des erfindungsgemäßen Verfahrens wird die verdünnte Schwefelsäure aus der Chlorgastrocknung aus Schritt g) und die verdünnte Schwefelsäure aus dem Nitrierverfahren gemäß Schritt b) in einer einzigen Schwefelsäureaufkonzentrierungsanlage vereinigt und aufbereitet und die aufkonzentrierte Schwefelsäure wieder anteilig in das Nitrierverfahren nach Schritt a) und die Chlortrocknung nach Schritt g) zurückgeführt oder einer weiteren Nitrieranlage, die eine andere aromatische Nitroverbindung als die erste Nitrieranlage herstellt, zugeführt. Diese besondere Ausführungsform des Verfahrens ermöglicht, dass mit einer einzigen Schwefelsäureaufkonzentrationsanlage sowohl Chlor getrocknet als auch Dinitrotoluol in der ersten Nitrieranlage und Nitrobenzol in der zweiten Nitrieranlage hergestellt werden kann, wobei für beide Nitrieranlagen die verdünnte Schwefelsäure, die aus dem Chlortrocknungsverfahren erhältlich ist gegebenenfalls vor dem Aufkonzentrierungsschritt j) als Katalysator in beide Nitrierungen eingesetzt werden kann. Diese Verfahrensalternative ist dann vorteilhaft, wenn die verdünnte Schwefelsäuremenge aus der Chlortrocknung die Schwefelsäureverluste einer der Nitrierungen übersteigt. Hierdurch wird wiederum der Einsatz von frischer Schwefelsäure in dem Chlortrocknungsschritt g), um den zurückgeführten Anteil vermindert.

Da für die Katalyse der Nitrierreaktion und die Chlortrocknung unterschiedliche Schwefelsäurekonzentrationen erforderlich sein können, kann die Schwefelsäure in mehreren Stufen aufkonzentriert werden, um die jeweils geeignete Schwefelsäurekonzentration in den jeweils erforderlichen Mengen zur Verfügung zu stellen. So kann beispielsweise für die Nitrierung von Toluol eine Schwefelsäurekonzentration von 86- 91 % Massenanteile Schwefelsäure bezogen auf Massenanteile Schwefelsäure und Wasser, wie in EP-A 0903 336 genannt, erforderlich sein während eine für die Chlortrocknung vorteilhafte höhere Konzentration von 96,0-98,0 % Massenanteile Schwefelsäure bezogen auf Massenanteile Schwefelsäure und Wasser, benötigt wird. In diesem Fall kann man das erfindungsgemäße Verfahren so ausführen, dass in der Schwefelsäureaufkonzentrieranlage der gesamte Strom auf die für die Nitrierung erforderliche Konzentration konzentriert wird und nur der für die Chlortrocknung erforderliche Teilstrom in einer zusätzlichen Destillationsstufe weiter aufkonzentriert wird. Auf diese Weise wird auch in diesem Fall das Ziel einer Verringerung der Investitionskosten erreicht.

Vor der Rückführung von Schwefelsäure aus einer gemeinsamen Säureaufkonzentrierungsstufe in die Chlorgastrocknung können gegebenenfalls Reinigungsschritte zur Entfernung von möglicherweise im Chlorherstellungsverfahren störenden Komponenten wie beispielsweise organische Nitroverbindungen, Benzoesäuren, nitrose Gase oder Nitrosylschwefelsäure durchgeführt werden. Dies kann mit Hilfe von Adsorptions- oder Strippverfahren erfolgen.

Vorteilhaft ist das erfindungsgemäße Verfahren, wenn als aromatische Ausgangsverbindung Toluol oder Benzol eingesetzt werden. So kann im Fall des Toluols nach zweifacher Nitrierung Dinitrotoluol gemäß Schritt a) erhalten werden, das anschließend nach den aus dem Stand der Technik bekannten Verfahren mit Wasserstoff zu Toluylendiamin (TDA) gemäß Schritt c1) umgesetzt werden und anschließend nach den aus dem Stand der Technik bekannten Verfahren mit Phosgen zu Toluylendiisocyanat (TDn gemäß Schritt d) umgesetzt werden kann.

Für die zweifache Nitrierung zur Herstellung von Dinitrotoluol wird in der zweiten Nitrierstufe, die vom Mononitrotoluol zum Dinitrotoluol führt üblicherweise eine Schwefelsäurekonzentration im Bereich von 86,0 bis 96,0 % Massenanteile Schwefelsäure bezogen auf Masseanteile Schwefelsäure und Wasser, vor der Salpetersäurezugabe eingesetzt. Diese Schwefelsäure wird durch während der Nitrierung entstehendes Reaktionswasser anschließend auf eine Konzentration von 80,0-85,9 Massenanteile Schwefelsäure bezogen auf Masseanteile Schwefelsäure und Wasser, verdünnt. Die aus dieser zweiten Nitrierungsstufe erhaltene verdünnte Schwefelsäure mit dem Konzentrationsbereich von 80,0-85,9 % Massenanteile Schwefelsäure bezogen auf Masseanteile Schwefelsäure und Wasser wird anschließend als Zulauf-Schwefelsäure für die erste Nitrierstufe vom Toluol zum Mononitrotoluol eingesetzt, wobei im Verlauf dieser ersten Nitrierstufe diese eingesetzte Schwefelsäure auf eine Konzentration von 70,0-79,9 % Massenanteile Schwefelsäure bezogen auf die Masseanteile Schwefelsäure und Wasser, verdünnt wird.

Im Falle des Benzols wird durch einfach Nitrierung nach Schritt a) des erfindungsgemäßen Verfahrens Nitrobenzol erhalten, das anschließend nach den aus dem Stand der Technik bekannten Verfahren mit Wasserstoff zu Anilin gemäß Schritt c2) des erfmdungsgemäßen Verfahrens umgesetzt werden, welches wiederum mit Formaldehyd in Gegenwart eines sauren Katalysators zu Di- und Polyaminen der Diphenylmethanreihe gemäß Schritt c2) umgesetzt wird. Die Di- und Polyaminen können anschließend nach den aus dem Stand der Technik bekannten Verfahren mit Phosgen zu den entsprechenden Di- und Polyisocyanaten der Diphenylmethanreihe gemäß Schritt d) umgesetzt werden.

Bei der Nitrierung von Benzol wird vor Salpetersäurezugabe eine Schwefelsäure eingesetzt, die eine Konzentration im Bereich von 69,5-72,5 % Massenanteile Schwefelsäure bezogen auf Masseanteile Schwefelsäure und Wasser, aufweist. Nach erfolgter Nitrierung weist die aus dieser Nitrierung erhaltene Schwefelsäure eine Konzentration von 66,5-69,4 % Massenanteile Schwefelsäure bezogen auf Massenanteile Schwefelsäure und Wasser auf.

Nach dem erfindungsgemäßen Verfahren erhaltenes Chlor kann anschließend nach den aus dem Stand der Technik bekannten Verfahren mit Kohlenmonoxid zu Phosgen gemäß Schritt h) umgesetzt werden, welches für die Herstellung von TDI oder MDI aus TDA beziehungsweise MDA aus Schritt c1) oder Schritt c2) erhältlich ist, eingesetzt werden kann. Der bei der Phosgenierung von TDA und MDA wiederum entstehende Chlorwasserstoff kann anschließend gemäß Schritt i) nach den aus dem Stand der Technik bekannten Verfahren zu Chlor gemäß Schritt f) umgesetzt werden.

## Patentansprüche

1. Verfahren zur gekoppelten Herstellung von Isocyanaten und Chlor umfassend folgende Schritte
a) Nitrierung von aromatischen Verbindungen ausgewählt aus der Gruppe von Toluol, halogeniertem Toluol, Benzol und halogeniertem Benzol zu den entsprechenden einfach oder zweifach nitrierten aromatischen Verbindungen unter Einsatz von Salpetersäure, wobei die Nitrierung in Abhängigkeit von der zu nitrierenden Verbindung in Gegenwart einer Schwefelsäure mit einer Konzentration von 65,0 - 98,0 % Massenanteile Schwefelsäure bezogen auf Massenanteile Schwefelsäure und Wasser als Katalysator durchgeführt wird, wobei die Schwefelsäure nach erfolgter Nitrierung verdünnt ist und eine Konzentration aufweist, die je nach eingesetzter Schwefelsäuremengc und Zahl der Nitrierstufen um 0,5 bis 25 Prozentpunkte niedriger ist als die eingesetzte Schwefelsäure.
b) Überführung der aus dem Schritt a) erhaltenen verdünnten Schwefelsäure in eine Schwefelsäureaufkonzentrieranlage,
c1) Umsetzung der aus Schritt a) erhaltenen einfach oder zweifach nitrierten aromatischen Verbindung mit Wasserstoff zu dem entsprechendem Amin, oder
c2) für den Fall, dass die einfach oder zweifach nitrierte aromatische Verbindung Nitrobenzol ist, Umsetzung des aus Schritt a) erhaltenen Nitrobenzols mit Wasserstoff zu Anilin und anschließende Umsetzung dieses Produktes mit Formaldehyd zu den Di- und Polyaminen der Diphenylmethanreihe,
d) Umsetzung der Amine erhältlich aus Schritt c1) oder c2) mit Phosgen zu den entsprechenden Isocyanaten,
e) Rückführung des bei der Reaktion der Amine aus Schritt c1) oder c2) mit Phosgen entstehenden wässrig gelösten oder gasförmigen Chlorwasserstoffs aus Schritt d) in Schritt f);
f) Herstellung von Chlorgas
g) Entfernung des Reaktionswassers oder aus anderen Quellen enthaltene Feuchtigkeit unter Trocknung aus dem aus Schritt f) erhaltenen Chlorgas durch Behandlung des aus Schritt f) erhaltenen Chlorgases mit einer Schwefelsäure mit einer Konzentration von 90,0 bis 99,0 % Massenanteile Schwefelsäure bezogen auf Massenanteile Schwefelsäure und Wasser bis die Schwefelsäure eine Konzentration von 65,0-90,0 % Massenanteile Schwefelsäure bezogen auf Massenanteile Schwefelsäure und Wasser aufweist,
h) Umsetzung des aus Schritt g) erhaltenen, trockenen Chlorgases mit Kohlenmonoxid zur Herstellung von Phosgen,
i) Einsatz des aus Schritt h) erhaltenen Phosgens in den Schritt d),
j) Überführung der aus Schritt g) erhaltenen verdünnten Schwefelsäure entweder in die gleiche Schwefelsäureaufkonzentrieranlage von Schritt b) oder direkt in einen oder mehrere Nitrierschritte gemäß Schritt a) und anschließende Überführung der aus dieser oder diesen Nitrierungen ablaufenden, weiter verdünnten Schwefelsäuren in die gleiche Schwefelsäureaufkonzentrieranlage gemäß Schritt b),
wobei die verdünnten Schwefelsäureströme aus Schritt b) und g) in der Schwefelsäureaufkonzentrieranlage vereint werden und durch eine oder mehrere Vakuumdestillationsstufen auf eine erhöhte Konzentration oder durch Abnahme von Teilströmen nach verschiedenen Destillationsstufen auf mehrere verschiedene erhöhte Konzentrationen angehoben wird, wie sie jeweils für einen oder mehrere der Verfahrensschritte der Aromatennitrierung nach Schritt a) oder Chlorgastrocknung nach Schritt g) benötigt werden
k) und anschließende Rückführung der aus Schritt j) gewonnenen aufkonzentrierten Schwefelsäure entweder zum vollständigem oder teilweisen Einsatz in Schritt g) und / oder Schritt a).

2. Verfahren nach Anspruch 1 bei dem die Herstellung des Chlorgas gemäß Schritt f) nach Anspruch 1 nach einem oder mehreren der Verfahren ausgewählt aus der Gruppe von Na-CI-Elektrolyse, HCl-Elektrolyse nach Membran- oder Diaphragma- Verfahren, HCl-Elektrolyse in Elektrolysezellen mit Sauerstoffverzehrkathode und katalytische HCl-Oxidation mit Sauerstoff erfolgt.

3. Verfahren nach einem der beiden Ansprüche 1 oder 2 , wobei die einfach oder zweifach nitrierten aromatischen Verbindungen ausgewählt sind aus der Gruppe von Nitrobenzol, Dinitrobenzol, Nitrochlorbenzol, Nitrochlortoluol, Nitrotoluol und Dinitrotoluol.

4. Verfahren nach einem der Ansprüche 1 bis 3 bei dem die verdünnte Schwefelsäure, die nach dem Verfahrensschritt g) erhalten wird vor Vereinigung in der Schwefelsäureaufkonzentrierungsanlage gemäß Schritt j) von Chlor- und/ oder HCl-Resten bis auf eine Restkonzentration von < 1000ppm Cl befreit wird.

5. Verfahren nach einem der Ansprüche 1 bis 4, bei dem die verdünnte Schwefelsäure, die nach dem Verfahrensschritt g) erhalten wird vor Vereinigung in der Schwefelsäureaufkonzentrierungsanlage gemäß Schritt j) von Chlor- und/ oder HCl- Resten bis auf eine Restkonzentration von < 10 ppm Cl befreit wird.

6. Verfahren nach einem der Ansprüche 1 bis 5 bei dem entweder bereits die verdünnte Schwefelsäure erhältlich nach Schritt a) oder erst die aufkonzentrierte Schwefelsäure erhältlich nach Schritt j) nach Anspruch 1 vor Rücküberführung in Schritt k) des Verfahrens nach Anspruch 1 von Verunreinigungen durch anorganische Stickstoffverbindungen wie Nitrosylschwefelsäure bis auf eine Restkonzentration von < 0,3 Gew.- % bezogen auf die Gesamtmasse oder durch leichtflüchtige organische Verbindungen wie Dinitrotoluol, Nitrobenzol, Dinitrobenzol, Nitrophenole, Nitrokresole, Nitrobenzylalkohole, Nitrobenzaldehyde bis auf eine Restkonzentration von < 50 ppm, oder durch schwer flüchtige organische Verbindungen wie Hydroxynitrobenzoesäure, Nitrobenzoesäuren oder aliphatische Carbonsäuren bis auf eine Restkonzentration von < 500 ppm oder durch flüchtige Schwefelverbindungen wie SO₂ bis auf eine Restkonzentration von < 30 ppm befreit wird.

7. Verfahren nach einem der Ansprüche 1 bis 6 bei dem in Schritt a) Toluol eingesetzt wird und nach zweifacher Nitrierung in Schritt a) Dinitrotoluol erhalten wird, wobei im ersten Schritt der Nitrierung eine Schwefelsäure mit einer Konzentration von 86,0-96,0 % Massenanteile Schwefelsäure bezogen auf die Summe der Massenanteile von Schwefelsäure und Wasser, eingesetzt wird, die im Laufe der zweiten Nitrierungsstufe auf eine Konzentration von 80,0-85,9 % Massenanteile Schwefelsäure bezogen auf die Summe der Massenanteile von Schwefelsäure und Wasser, verdünnt wird, das Dinitrotoluol anschließend gemäß Schritt c1) mit Wasserstoff zu Toluylendiamin (TDA) umgesetzt wird, welches anschließend mit Phosgen gemäß Schritt d) zu Toluylendiisocyanat (TDI) umgesetzt wird.

8. Verfahren nach Anspruch 7, wobei die Schwefelsäure, die aus der zweiten Nitrierstufe mit einer Konzentration von 80,0-85,9 % Massenanteile Schwefelsäure bezogen auf die Masseanteile an Schwefelsäure und Wasser, erhalten wird, noch einmal in die erste Nitrierstufe des Verfahrens nach Schritt a) zurückgeleitet wird und anschließend auf eine Konzentration von 70,0-79,9 % Massenanteile Schwefelsäure bezogen auf die Masseanteile Schwefelsäure und Wasser, verdünnt wird und diese Schwefelsäure anschließend in die Schwefelsäureaufkonzentrierungsanlage gemäß Schritt b) geleitet wird.

9. Verfahren nach einem der Ansprüche 1 bis 6 bei dem in Schritt a) Benzol eingesetzt wird und nach einfacher Nitrierung in Schritt a) Nitrobenzol erhalten wird, wobei zur Nitrierung eine Schwefelsäure mit einer Konzentration von 69,5-72,5 % Massenanteile Schwefelsäure bezogen auf die Masseanteile Schwefelsäure und Wasser, eingesetzt wird, die im Laufe der Nitrierung auf eine Konzentration von 66,5-69,4 % Massenanteile Schwefelsäure bezogen auf die Masseanteile Schwefelsäure und Wasser, verdünnt wird, das Nitrobenzol anschließend gemäß Schritt c2) mit Wasserstoff zu Anilin umgesetzt wird, welches wiederum mit Formaldehyd in Gegenwart eines sauren Katalysators zu Di- und Polyaminen der Diphenylmethanreihe umgesetzt wird, welche wiederum anschließend mit Phosgen gemäß Schritt d) zu den entsprechenden Di- und Polyisocyanaten der Diphenylmethanreihe umgesetzt werden.

## Claims

1. Process for the coupled production of isocyanates and chlorine comprising the following steps:
a) Nitration of aromatic compounds selected from the group comprising toluene, halogenated toluene, benzene and halogenated benzene to form the corresponding mono- or dinitrated aromatic compounds using nitric acid, the nitration being performed, depending on the compound to be nitrated, in the presence of a sulfuric acid having a concentration of 65.0 to 98.0 percent by mass of sulfuric acid based on the percent by mass of sulfuric acid and water as catalyst, wherein on completion of nitration the sulfuric acid is diluted and has a concentration that, depending on the amount of sulfuric acid used and the number of nitration stages, is 0.5 to 25 percentage points lower than that of the sulfuric acid used,
b) Transfer of the dilute sulfuric acid obtained from step a) to a sulfuric acid concentration plant,
c1) Reaction of the mono- or dinitrated aromatic compound obtained from step a) with hydrogen to form the corresponding amine, or
c2) if the mono- or dinitrated aromatic compound is nitrobenzene, reaction of the nitrobenzene obtained from step a) with hydrogen to form the aniline and subsequent reaction of this product with formaldehyde to form the diamines and polyamines of the diphenylmethane series,
d) Reaction of the amines obtainable from step c1) or c2) with phosgene to form the corresponding isocyanates,
e) Return of the aqueously dissolved or gaseous hydrogen chloride formed in the reaction of the amines from step c1) or c2) with phosgene from step d) to step f),
f) Production of chlorine gas,
g) Removal of the reaction water or moisture contained from other sources with drying of the chlorine gas obtained from step f) by treating the chlorine gas obtained from step f) with a sulfuric acid having a concentration of 90.0 to 99.0 percent by mass of sulfuric acid based on the percent by mass of sulfuric acid and water until the sulfuric acid has a concentration of 65.0 to 90.0 percent by mass of sulfuric acid based on the percent by mass of sulfuric acid and water,
h) Reaction of the dry chlorine gas obtained from step g) with carbon monoxide to produce phosgene,
i) Use of the phosgene obtained from step h) in step d),
j) Transfer of the dilute sulfuric acid obtained from step g) either to the same sulfuric acid concentration plant from step b) or directly to one or more nitration stages according to step a) and subsequent transfer of the further diluted sulfuric acids obtained from this or these nitration(s) to the same sulfuric acid concentration plant according to step b),
wherein the dilute sulfuric acid streams from step b) and g) are combined in the sulfuric acid concentration plant and raised to an elevated concentration by means of one or more vacuum distillation stages or raised to several different elevated concentrations by removing split streams after different distillation stages, as required for one or more of the process stages of aromatic nitration according to step a) or chlorine gas drying according to step g)
k) and subsequent return of the concentrated sulfuric acid obtained from step j) for either full or partial use in step g) and/or step a).

2. Process according to claim 1 wherein the production of chlorine gas according to step f) of claim 1 takes place by one or more of the processes selected from the group comprising NaCl electrolysis, HCl electrolysis by membrane or diaphragm methods, HCl electrolysis in electrolytic cells with an oxygen depletion cathode and catalytic HCl oxidation with oxygen.

3. Process according to one of claims 1 or 2, wherein the mononitrated or dinitrated aromatic compounds are selected from the group comprising nitrobenzene, dinitrobenzene, nitrochlorobenzene, nitrochlorotoluene, nitrotoluene and dinitrotoluene.

4. Process according to one of claims 1 to 3 wherein the dilute sulfuric acid obtained by process step g) is freed from residual chlorine and/or HCl down to a residual concentration of < 1000 ppm Cl before combination in the sulfuric acid concentration plant according to step j).

5. Process according to one of claims 1 to 4 wherein the dilute sulfuric acid obtained by process step g) is freed from residual chlorine and/or HCl down to a residual concentration of < 10 ppm Cl before combination in the sulfuric acid concentration plant according to step j).

6. Process according to one of claims 1 to 5 wherein, before being returned to step k) of the process according to claim 1, either the dilute sulfuric acid obtainable by step a) or the concentrated sulfuric acid obtainable by step j) according to claim 1 is freed from impurities using inorganic nitrogen compounds such as nitrosyl sulfuric acid down to a residual concentration of < 0.3 wt.% based on the total mass or using highly volatile organic compounds such as dinitrotoluene, nitrobenzene, dinitrobenzene, nitrophenols, nitrocresols, nitrobenzyl alcohols, nitrobenzaldehydes down to a residual concentration of < 50 ppm, or using low-volatility organic compounds such as hydroxynitrobenzoic acid, nitrobenzoic acids or aliphatic carboxylic acids down to a residual concentration of < 500 ppm or using volatile sulfur compounds such as SO₂ down to a residual concentration of < 30 ppm.

7. Process according to one of claims 1 to 6 wherein toluene is used in step a) and after dinitration in step a) dinitrotoluene is obtained, a sulfuric acid with a concentration of 86.0 to 96.0 percent by mass of sulfuric acid based on the total percent by mass of sulfuric acid and water being used in the first nitration stage, which in the course of the second nitration stage is diluted to a concentration of 80.0 to 85.9 percent by mass of sulfuric acid based on the total percent by mass of sulfuric acid and water, the dinitrotoluene is then reacted according to step c1) with hydrogen to form toluylene diamine (TDA) which is then reacted with phosgene according to step d) to form toluylene diisocyanate (TDI).

8. Process according to claim 7, wherein the sulfuric acid obtained from the second nitration stage with a concentration of 80.0 to 85.9 percent by mass of sulfuric acid based on the percent by mass of sulfuric acid and water is returned once more to the first nitration stage of the process according to step a) and then diluted to a concentration of 70.0 to 79.9 percent by mass of sulfuric acid based on the percent by mass of sulfuric acid and water and this sulfuric acid is then fed to the sulfuric acid concentration plant according to step b).

9. Process according to one of claims 1 to 6 wherein benzene is used in step a) and after mononitration in step a) nitrobenzene is obtained, a sulfuric acid with a concentration of 69.5 to 72.5 percent by mass of sulfuric acid based on the percent by mass of sulfuric acid and water being used for nitration, which in the course of nitration is diluted to a concentration of 66.5 to 69.4 percent by mass of sulfuric acid based on the percent by mass of sulfuric acid and water, the nitrobenzene is then reacted according to step c2) with hydrogen to form aniline, which in turn is reacted with formaldehyde in the presence of an acid catalyst to form diamines and polyamines of the diphenylmethane series, which in turn are then reacted with phosgene according to step d) to form the corresponding di- and polyisocyanates of the diphenylmethane series.

## Revendications

1. Procédé pour la préparation conjuguée d'isocyanates et de chlore, comprenant les étapes suivantes :
a) nitration de composés aromatiques choisis parmi le groupe du toluène, du toluène halogéné, du benzène et du benzène halogéné, en les composés aromatiques nitrés une ou deux fois, avec mise en oeuvre d'acide nitrique, où la nitration est réalisée en fonction du composé à nitrer, en présence d'acide sulfurique en une concentration de 65,0-98,0% en masse d'acide sulfurique sur base de la quantité en masse d'acide sulfurique et d'eau comme catalyseur, où l'acide sulfurique est dilué après la nitration et présente une concentration, qui selon la quantité d'acide sulfurique mise en oeuvre et le nombre d'étapes de nitration, est plus faible d'environ 0,5 à 25% que l'acide sulfurique mis en oeuvre,
b) transfert de l'acide sulfurique dilué obtenu en a) dans une installation de concentration de l'acide sulfurique,
c1) réaction du composé aromatique nitré une ou deux fois obtenu en a), avec de l'hydrogène en l'amine correspondante, ou
c2) dans le cas où le composé aromatique nitré une ou deux fois est le nitrobenzène, réaction du nitrobenzène obtenu en a) avec l'hydrogène en aniline et ensuite, réaction de ce produit avec le formaldéhyde en les di- et polyamines de la série du diphénylméthane,
d) réaction de l'amine obtenue à l'étape c1) ou c2) avec le phosgène en les isocyanates correspondants,
e) recyclage du chlorure d'hydrogène dissous dans l'eau ou gazeux, formé lors de la réaction de l'amine de l'étape c1) ou c2) avec le phosgène, de l'étape d) à l'étape f),
f) préparation de chlore gazeux,
g) élimination de l'eau de réaction ou de l'humidité provenant d'autres sources par séchage du chlore gazeux obtenu à l'étape f), par traitement du chlore gazeux obtenu à l'étape f) avec de l'acide sulfurique ayant une concentration allant de 90,0 à 99,0% en masse d'acide sulfurique sur base de la quantité en masse d'acide sulfurique et d'eau, jusqu'à ce que l'acide sulfurique présente une concentration en acide sulfurique de 65,0-90,0% en masse sur base de la quantité en masse d'acide sulfurique et d'eau,
h) réaction du chlore gazeux séché, obtenu à l'étape g), avec le monoxyde de carbone pour la préparation du phosgène,
i) mise en oeuvre du phosgène obtenu à l'étape h) dans l'étape d),
j) transfert de l'acide sulfurique dilué obtenu à l'étape g), dans la même installation de concentration d'acide sulfurique que l'étape b) ou dans une ou plusieurs étapes de nitration selon l'étape a), et ensuite, transfert de l'acide sulfurique dilué, découlant de cette ou de ces étapes de nitration, dans la même installation de concentration d'acide sulfurique que l'étape b),
où les courants d'acide sulfurique dilué des étapes b) et g) sont rassemblés dans l'installation de concentration d'acide sulfurique, et par une ou plusieurs étapes de distillation sous vide, on relève à une concentration plus élevée, ou par extraction de courants partiels après différentes étapes de distillation, on relève à plusieurs concentrations plus élevées différentes, comme elles sont nécessaires pour une ou plusieurs des étapes de procédé de nitration d'aromatique selon l'étape a) ou de séchage du chlore gazeux selon l'étape g),
k) et ensuite, recyclage de l'acide sulfurique concentré obtenu à l'étape j), pour une mise en oeuvre complète ou partielle dans l'étape g) et/ou l'étape a).

2. Procédé selon la revendication 1, dans lequel la préparation du chlore gazeux selon l'étape f) de la revendication 1 est réalisée selon un ou plusieurs procédés choisis parmi le groupe de l'électrolyse du NaCl, l'électrolyse de HCl par des procédés à membrane ou à diaphragme, l'électrolyse de HCl dans des cellules d'électrolyse avec cathode à consommation d'oxygène et oxydation catalytique de HCl avec l'oxygène.

3. Procédé selon l'une des revendications 1 ou 2, dans lequel les composés aromatiques nitrés une ou deux fois sont choisis dans le groupe du nitrobenzène, du dinitrobenzène, du nitrochlorobenzène, du nitrochlorotoluène, du nitrotoluène et du dinitriotoluène.

4. Procédé selon l'une des revendications 1 à 3, dans lequel l'acide sulfurique dilué, qui est obtenu après l'étape g), avant rassemblement dans l'installation de concentration d'acide sulfurique selon l'étape j), est libéré des restes de chlore et/ou de HCl jusqu'à une concentration résiduelle de < 1000 ppm en Cl.

5. Procédé selon l'une des revendications 1 à 4, dans lequel l'acide sulfurique dilué, qui est obtenu après l'étape g), avant rassemblement dans l'installation de concentration d'acide sulfurique selon l'étape j), est libéré des restes de chlore et/ou de HCl jusqu'à une concentration résiduelle de < 10 ppm en Cl.

6. Procédé selon l'une des revendications 1 à 5, dans lequel l'acide sulfurique déjà dilué obtenu après l'étape a), ou l'acide sulfurique concentré obtenu après l'étape j) selon la revendication 1, est libéré avec recyclage dans l'étape k) du procédé selon la revendication 1, des impuretés par des composés azotés inorganiques, comme l'acide nitrosylsulfurique, jusqu'à une concentration résiduelle de < 0,3% en poids sur base de la masse totale ou par des composés organiques volatils comme le dinitrotoluène, le nitrobenzène, le dinitrobenzène, les nitrophénols, les nitrocrésols, les nitrobenzylalcools, le nitrobenzaldéhyde, jusqu'à une concentration résiduelle de < 50 ppm, ou par des composés organiques peu volatils comme l'acide hydroxynitrobenzoïque, les acides nitrobenzoïques ou des acides carboxyliques aliphatiques, jusqu'à une concentration résiduelle de < 500 ppm, ou par des composés soufrés volatils comme SO₂, jusqu'à une concentration résiduelle de < 30 ppm.

7. Procédé selon l'une des revendications 1 à 6, dans lequel dans l'étape a), le toluène est mis en oeuvre et après nitration double dans l'étape a), on obtient le dinitrotoluène, où dans une première étape de la nitration, un acide sulfurique ayant une concentration de 86,0-96,0% en masse d'acide sulfurique sur base de la somme des quantités en masse d'acide sulfurique et d'eau, est mis en oeuvre, qui est dilué au cours de la deuxième étape de nitration, à une concentration de 80,0-85,9% en masse d'acide sulfurique sur base de la somme des quantités en masse d'acide sulfurique et d'eau, le dinitrotoluène est ensuite mis à réagir avec l'hydrogène selon l'étape c1), en tolyulènediamine (TDA), qui est mis à réagir ensuite avec le phosgène selon l'étape d), en toluylènediisocyanate (TDI).

8. Procédé selon la revendication 7, dans lequel l'acide sulfurique, qui est obtenu de la deuxième étape de nitration avec une concentration de 80,0-85,9% en masse d'acide sulfurique sur base des quantités en masse d'acide sulfurique et d'eau, et ramené encore une fois dans la première étape de nitration du procédé selon l'étape a) et ensuite, est dilué à une concentration de 70,0-79,9% en masse d'acide sulfurique sur base des quantités en masse d'acide sulfurique et d'eau, et cet acide sulfurique est ensuite dirigé dans l'installation de concentration d'acide sulfurique selon l'étape b).

9. Procédé selon l'une des revendications 1 à 6, dans lequel dans l'étape a), le benzène est mis en oeuvre et après nitration simple dans l'étape a), on obtient le nitrobenzène, où pour la nitration, un acide sulfurique ayant une concentration de 69,5-72,5% en masse d'acide sulfurique sur base des quantités en masse d'acide sulfurique et d'eau, est mis en oeuvre, qui est dilué au cours de la nitration, à une concentration de 66,5-69,4% en masse d'acide sulfurique sur base des quantités en masse d'acide sulfurique et d'eau, le nitrobenzène est ensuite mis à réagir selon l'étape c2), avec l'hydrogène en aniline, qui est mise à réagir ensuite avec le formaldéhyde en présence d'un catalyseur acide, en di- et polyamines de la série du diphénylméthane, qui sont mises à réagir ensuite, avec le phosgène selon l'étape d), en les di- et polyisocyanates de la série du diphénylméthane correspondants.
